# EUROPÄISCHE PATENTANMELDUNG

(11) **EP 4 483 805 A1**
(43) Veröffentlichungstag der Anmeldung: **01.01.2025**
(21) Anmeldenummer: 24183282.3
(22) Anmeldetag: 20.06.2024
(51) Int. Cl.: A61B 5/1455

(54) **SAUERSTOFFSÄTTIGUNGSSENSOR**

(30) Priorität: 29.06.2023 DE 102023117130
(71) Anmelder: Drägerwerk AG & Co. KGaA, 23558 Lübeck (DE)
(72) Erfinder: Graßl, Thomas, 23558 Lübeck (DE); Hoot, Martha, 23558 Lübeck (DE)

(57) **Zusammenfassung**

Erfindungsgemäß wird ein Sauerstoffsättigungssensor bereitgestellt. Der Sauerstoffsättigungssensor umfasst eine sich in Richtung einer Längsachse erstreckende textile Grundstruktur, wobei die Grundstruktur eine rückstellend veränderliche Querschnittsform aufweist, so dass der Sauerstoffsättigungssensor auf einer Messstelle fixierbar ist. Der Sauerstoffsättigungssensor umfasst ferner eine lichtemittierende Diode und einen Strahlungsdetektor. Die lichtemittierende Diode und der Strahlungsdetektor sind in oder an der Grundstruktur aufgenommen und so in einer Querschnittsebene der Grundstruktur angeordnet.

## Beschreibung

Die vorliegende Erfindung betrifft einen Sauerstoffsättigungssensor, insbesondere einen pulsoxymetrischen Sauerstoffsättigungssensor.

Sauerstoffsättigungssensoren dienen zur Ermittlung der Sättigung des Blutes mit Sauerstoff (Sauerstoffsättigung). Eine spezielle Ausgestaltung eines Sauerstoffsättigungssensors ist ein pulsoxymetrischer Sauerstoffsättigungssensor.

Sauerstoffsättigungssensoren werden beispielsweise in der Intensivpflege und Anästhesie eingesetzt, wobei es üblich ist, dass die Messung der Sauerstoffsättigung kontinuierlich über einen längeren Zeitraum durchgeführt wird.

Ein Sauerstoffsättigungssensor weist wenigstens eine lichtemittierende Diode (LED), beispielsweise eine rote LED und/oder eine infrarote LED sowie wenigstens einen Strahlungsdetektor, beispielsweise eine Photodiode auf. Zur Ermittlung der Sauerstoffsättigung kann eine Messung und Auswertung der Absorptionseigenschaften oder Transmissionseigenschaften der Messstelle, beispielsweise eines Fingers eines Benutzers, in Bezug auf das von der wenigstens einen LED abgestrahlte Licht erfolgen.

Abhängig vom gewünschten Anwendungszweck können Sauerstoffsättigungssensoren wiederverwendbar oder zur einmaligen Verwendung ausgestaltet sein.

Ein aus DE 3 703 458 A1 bekannter wiederverwendbarer Sensor weist einen durch Materialdehnung deformierbaren Trägerkörper aus Silikon, Kautschuk oder Polyurethan auf, welcher über einen Finger, Arm oder Bein eines Patienten gelegt werden kann und dort durch Klemmkräfte haftet. Der aus DE 3 703 458 A1 bekannte Sensor ist aufgrund des komplex ausgestalteten Trägerkörpers aufwändig zu reinigen und zu desinfizieren.

Ein aus DE 69 117 861 T2 bekannter Sensor weist einen wiederverwendbaren Sensorteil auf, welcher eine Photodiode umfasst und welcher auf einem Finger eines Patienten positioniert werden kann. Der Sensor weist ferner ein wegwerfbares flexibles Element aus Vlies auf, welches durch eine Klebeschicht am Finger fixiert werden kann und einen Photoemitter umfasst.

Ein aus US 6 073 038 A bekannter Oximetriesensor weist ein Schaumstoffumhüllungselement mit einem Befestigungselement, ein Rückseitenfolienbefestigungselement, eine LED-Baugruppe und eine mit einem Kabel verbundene Photodiode auf.

Eine aus CN 2 09 122 243 U bekannter Sauerstoffsättigungsmessvorrichtung umfasst eine Druckhülse. In einem ersten Ende der Druckhülse ist eine Trageöffnung ausgebildet, und ein Sauerstoffsättigungssensor ist an einer Innenwand der Druckhülse angeordnet und befindet sich in der Nähe eines zweiten Endes der Druckhülse. Die Druckhülse ist ausgebildet, um auf einem Finger einer zu erfassenden Person angeordnet zu werden. Die Druckhülse ist aus elastischem Gewebe hergestellt.

Aus US 2020 / 0 015 746 A1 ist eine tragbare Vorrichtung zur Überwachung eines oder mehrerer Vitalzeichen eines menschlichen Körper bekannt. Die Vorrichtung umfasst einen Träger, der geeignet ist, um einen Bauchteil eines Körpers getragen zu werden, und eine Elektrodenanordnung, die eine Vielzahl von leitenden Elektroden umfasst, wobei die Elektroden auf dem Träger so angeordnet sind, dass sie bei der Verwendung in Kontakt mit einer Haut des Körpers gebracht werden, und wobei die Elektroden so angeordnet sind, dass sie elektrische physiologische Signale vom Körper empfangen, um die Überwachung eines Membranpotentials eines oder mehrerer Muskeln im Körper zu ermöglichen.

Aus US 2020 / 0 297 279 A1 ist eine Manschette bekannt, die an einer Gliedmaße eines Benutzers getragen werden kann. Die Manschette kann verwendet werden, um Form, Position und Bewegung der Gliedmaße sowie biometrische Eigenschaften des Benutzers zu erfassen.

Nachteilhaft an den aus DE 3 703 458 A1 und DE 69 117 861 T2 bekannten Sensoren ist, dass die Ausrichtung von Photodiode und Photoemitter zueinander abhängig von der Größe des in dem Sensor aufgenommenen Fingers ist. Dies kann zu einem nachteilhaften Signal-Rausch-Verhältnis führen. Zudem muss zur Vermeidung von Langzeiteffekten wie Druckstellen an dem Finger eines Benutzers die Position des Sensors regelmäßig verändert werden, was bei dem Sensor nach DE 69 117 861 T2 ein Lösen und vollständiges Austauschen des flexiblen Elements erforderlich macht. Hierdurch steigen die Betriebskosten eines derartigen Sensors nachteilhaft an.

Aufgabe der vorliegenden Erfindung ist es, einen Sauerstoffsättigungssensor bereitzustellen, welcher diese Nachteile wenigstens teilweise nicht aufweist und insbesondere einen Sauerstoffsättigungssensor bereitzustellen, dessen Messeigenschaften und Nutzerkomfort verbessert sind.

Diese und weitere Aufgaben werden gelöst durch den Gegenstand des unabhängigen Patentanspruchs.

Die abhängigen Ansprüche, Beschreibung und Figuren stellen vorteilhafte Ausgestaltungen und weitere Details der Erfindung bereit.

Erfindungsgemäß wird ein Sauerstoffsättigungssensor bereitgestellt. Der Sauerstoffsättigungssensor umfasst eine sich in Richtung einer Längsachse erstreckende textile Grundstruktur, wobei die Grundstruktur eine rückstellend veränderliche Querschnittsform aufweist, so dass der Sauerstoffsättigungssensor auf einer Messstelle fixierbar ist. Der Sauerstoffsättigungssensor umfasst ferner eine lichtemittierende Diode und einen Strahlungsdetektor. Die lichtemittierende Diode und der Strahlungsdetektor sind in oder an der Grundstruktur aufgenommen und so in einer Querschnittsebene der Grundstruktur angeordnet.

Unter einem Sauerstoffsättigungssensor wird im Rahmen der Erfindung ein Sensor zur Ermittlung der Sättigung des Blutes mit Sauerstoff (Sauerstoffsättigung) verstanden. Vorzugsweise ist der Sauerstoffsättigungssensor als ein pulsoxymetrischer Sauerstoffsättigungssensor ausgestaltet.

Unter einer Grundstruktur wird eine Struktur verstanden, welche geeignet ist, eine lichtemittierende Diode und einen Strahlungsdetektor aufzunehmen und welche auf einer Messstelle fixierbar ist. Unter einer Messstelle wird ein Teil eines menschlichen Körpers verstanden, welcher zur Messung der Sauerstoffsättigung geeignet ist, beispielsweise ein Finger, ein Arm oder ein Bein.

Die Grundstruktur ist erfindungsgemäß als textile Grundstruktur ausgestaltet, worunter verstanden wird, dass die Grundstruktur als flächenförmiges oder raumförmiges textiles Gebilde ausgestaltet ist, d.h. ein oder mehrere flächenförmige oder raumförmige textile Gebilde umfasst oder aus solchen besteht. Die Grundstruktur kann prinzipiell eine beliebige Form aufweisen, es ist jedoch bevorzugt, dass die Grundstruktur in Bezug auf die Längsachse rotationssymmetrisch ausgebildet ist.

Unter einer rückstellend veränderlichen Querschnittsform wird verstanden, dass die Grundstruktur eine zur Aufnahme der Messstelle geeignete Querschnittsform aufweist, wobei die Grundstruktur bzw. deren Querschnittsform durch Krafteinwirkung aus ihrer Ursprungsform heraus veränderlich, insbesondere vergrößerbar, ist und wobei die Grundstruktur im deformierten Zustand eine Rückstellkraft ausübt, um die Querschnittsform aus dem deformierten Zustand zur Ursprungsform hin zu verändern, insbesondere zur Ursprungsform hin zu verkleinern. In anderen Worten ist die textile Grundstruktur elastisch deformierbar. Hierdurch erzeugt die Grundstruktur eine fixierende Klemmwirkung auf bzw. an der Messstelle, wenn die Messstelle größere Abmessungen aufweist als der Querschnitt in seiner Ursprungsform. Somit ist der Sauerstoffsättigungssensor durch die rückstellend veränderliche Querschnittsform auf der Messstelle reversibel fixierbar.

Für den Fall, dass die Grundstruktur entlang der Längsachse eine nichtgleichmäßig ausgestaltete Querschnittsform aufweist, bezeichnet die Querschnittsform die Querschnittsform, die in der Querschnittsebene vorliegt, in welcher die LED und der Strahlungsdetektor angeordnet sind.

Die Grundstruktur kann einen entlang sich entlang der Längsachse erstreckenden Hohlkörper definieren, welcher wenigstens in der Querschnittsebene die rückstellend veränderliche Querschnittsform aufweist. Der Hohlkörper kann beispielsweise zylindrisch, kegelstumpfförmig oder als Hyperboloid ausgestaltet sein, d.h. durch einen Zylinder, einen Kegelstumpf oder ein Hyperboloid als Begrenzungsfläche begrenzt sein.

Der Sauerstoffsättigungssensor kann eine oder mehrere lichtemittierende Dioden (im Folgenden auch bezeichnet als LED bzw. im Plural als LEDs) aufweisen. Vorzugsweise weist der Sauerstoffsättigungssensor eine rote LED und/oder eine infrarote LED auf. Unter einer roten LED wird eine LED verstanden, welche eingerichtet ist, Licht mit einer Wellenlänge im Bereich von 610 nm bis 760 nm, vorzugsweise mit einer Wellenlänge von 660 nm abzustrahlen. Unter einer infraroten LED wird eine LED verstanden, welche eingerichtet ist, Licht mit einer Wellenlänge von mehr als 760 nm, vorzugsweise in einem Bereich von 800 nm bis 1000 nm, besonders bevorzugt mit einer Wellenlänge von 950 nm abzustrahlen.

Falls der Sauerstoffsättigungssensor mehrere LEDs aufweist, können diese als Teil eines gemeinsamen Bauteils ausgestaltet sein oder als strukturell getrennte Bauteile ausgestaltet sein.

Unter einem Strahlungsdetektor wird ein Bauelement verstanden, welches zur Messung elektromagnetischer Strahlung, nämlich Licht eingerichtet ist. Ein solcher Strahlungsdetektor wird auch als Photodetektor bezeichnet. Der Strahlungsdetektor kann beispielsweise als Photowiderstand, Photodiode, Phototransistor, CCD-Sensor und/oder als CMOS-Sensor ausgestaltet sein. Bevorzugt ist der Strahlungsdetektor als Photodiode ausgestaltet.

Die LED und/oder der Strahlungsdetektor kann bzw. können eine oder mehrere Elemente zur Beeinflussung des Strahlengangs in Richtung zu oder von der LED und/oder in Richtung zu oder von dem Strahlungsdetektor aufweisen, wie beispielsweise eine oder mehrere Linsen und/oder einen oder mehrere Reflektoren.

Vorzugsweise schließen die Längsachse, die LED (bzw. jede der LEDs) und der Strahlungsdetektor (bzw. jeder der Strahlungsdetektoren) einen Winkel (bzw. jeweils einen Winkel) ein, der zwischen 90 und 270 Grad, besonders bevorzugt zwischen 90 Grad und 180 Grad beträgt.

Vorzugsweise ist der erfindungsgemäße Sauerstoffsättigungssensor als wegwerfbarer Sauerstoffsättigungssensor ausgestaltet. Alternativ ist bevorzugt, dass der Sauerstoffsättigungssensor als wiederverwendbarer Sauerstoffsättigungssensor ausgestaltet ist, wobei dieser durch die textile Grundstruktur einfach aufbereitet, d.h. gereinigt und desinfiziert werden kann. Beispielsweise ist eine Reinigung in einer Waschmaschine möglich.

Vorzugsweise ist die Querschnittsform durch Stauchen der Grundstruktur in Richtung der Längsachse rückstellend veränderlich, insbesondere rückstellend vergrößerbar. Auf diese Weise kann die Handhabung des Sauerstoffsättigungssensors verbessert werden, da der Sauerstoffsättigungssensor zum Aufsetzen auf die Messstelle durch Stauchen in seinem Durchmesser vergrößert und der Sauerstoffsättigungssensor somit ohne Zug- oder Druckbeanspruchung der Messstelle auf diese aufgesetzt werden kann. Durch anschließendes Entlasten der Grundstruktur verkleinert sich die Querschnittsform aufgrund der Rückstellwirkung wieder und erzeugt damit die fixierende Klemmwirkung auf der Messstelle.

In einer besonders bevorzugten Ausgestaltung ist die Querschnittsform durch Stauchen der Grundstruktur in Richtung der Längsachse rückstellend veränderlich, so dass die Querschnittsform (wenigstens im Bereich der LED und des Strahlungsdetektors, d.h. wenigstens in der Querschnittsebene) sowohl im gestauchten als auch im nicht gestauchten Zustand im Wesentlichen kreisförmig ist.

Alternativ vorzugsweise ist der Querschnitt durch Deformation der Grundstruktur quer zur Längsachse, insbesondere in der Querschnittsebene, rückstellend veränderlich. Auf diese Weise kann die Handhabung des Sauerstoffsättigungssensors verbessert werden, da der Sauerstoffsättigungssensor auf die Messstelle, beispielsweise auf einen Finger, aufgeschoben werden kann und durch dieses Aufschieben eine Deformation der Grundstruktur quer zur Längsachse eintritt, welche zur Rückstellung der Querschnittsform und folglich zur fixierenden Klemmwirkung führt. Eine zusätzliche manuelle Deformation der Grundstruktur zum Aufsetzen auf die Messstelle kann somit vorteilhaft entfallen.

Erfindungsgemäß umfasst die Grundstruktur eine Mehrzahl biegeelastischer Garne, wobei wenigstens ein Teil der Garne ausgewählt ist aus der Gruppe umfassend Kunststoffgarn, Glasgarn und Kohlenstoffgarn.

Unter einem Garn wird ein linienförmiges textiles Gebilde verstanden, welches eine oder mehrere Fasern umfasst. Vorzugsweise ist das biegeelastische Garn als Monofil-Garn ausgestaltet. Ein Kunststoffgarn bezeichnet daher ein Garn, welches eine oder mehrere Kunststofffasern umfasst oder aus einer oder mehreren Kunststofffasern besteht. Ein Glasgarn bezeichnet ein Garn, welches eine oder mehrere Glasfasern umfasst oder aus einer oder mehreren Glasfasern besteht. Ein Kohlenstoffgarn bezeichnet ein Garn, welches eine oder mehrere Kohlenstofffasern umfasst oder aus einer oder mehreren Kohlenstofffasern besteht.

Auf diese Weise kann die elastische Verformbarkeit der Grundstruktur vorteilhaft erhöht und somit die Fixierung des Sauerstoffsättigungssensors auf der Messstelle verbessert werden.

Besonders bevorzugt besteht die Grundstruktur aus einer Mehrzahl biegeelastischer Garne. Auf diese Weise kann die elastische Deformierbarkeit der Grundstruktur noch weiter erhöht werden.

Besonders bevorzugt umfasst die Grundstruktur eine Mehrzahl biegeelastischer Garne und eine Mehrzahl biegeschlaffer Garne.

Durch die Kombination biegeelastischer Garne und biegeschlaffer Garne kann eine Grundstruktur mit hoher elastischer Deformierbarkeit bereitgestellt werden, welche gleichzeitig einen verbesserten Tragekomfort aufweist, da biegeschlaffe, also weiche Garne im Allgemeinen angenehmer auf der Haut eines Patienten aufliegen. Ferner können so die Eigenschaften der Grundstruktur besser an die Einsatzbedingungen des Sauerstoffsättigungssensors angepasst werden.

Vorzugsweise ist das Material des Kunststoffgarns ausgewählt aus der Gruppe umfassend Polypropylen, Polyurethan und Polyethylen.

Kunststoffe aus dieser Gruppe weisen vorteilhafte mechanische Eigenschaften bei gleichzeitig geringen Kosten auf.

Erfindungsgemäß ist die Grundstruktur als geflochtene, gewickelte, gewebte oder gestrickte Röhre ausgebildet.

Unter einer Röhre wird eine entlang der Längsachse länglich ausgebildete Grundstruktur verstanden, welche im Inneren einen vorbeschriebenen Hohlkörper zur Aufnahme der Messstelle bereitstellt und im Wesentlichen die Form eines Zylinders, eines (ggf. Doppel-)Kegelstrumpfs oder eines Hyperboloids aufweist.

Unter einer geflochtenen, gewickelten, gewebten und/oder gestrickten Röhre wird verstanden, dass die Grundstruktur unter Nutzung eines Flechtverfahrens, Wickelverfahrens, Webverfahrens oder Strickverfahrens erhalten wird. Dabei kann die Röhre sowohl durch Bereitstellen einer oder mehrerer flächenförmiger textiler Gebilde und nachträglichem Fügen dieser zu einer Röhre als auch durch Bereitstellen eines (ggf. aus Teilelementen gefügten) raumförmigen textilen Gebildes erhalten werden. Ebenso können die vorstehend genannten Herstellverfahren kombiniert werden, um die Röhre zu erhalten. Beispielsweise kann eine gestrickte Röhre mit Einlegefäden so versehen sein, dass die Einlegefäden eine geflochtene Struktur ergeben. Ferner beispielsweise kann eine geflochtene Struktur ein mitgeführtes Garn aufweisen.

Geflochtene, gewickelte, gewebte und/oder gestrickte Röhren bieten den Vorteil, dass sie einfach zu fertigen sind und eine hohe Deformierbarkeit entlang der Längsachse und/oder quer zur Längsachse aufweisen.

Vorzugsweise kann so eine Grundstruktur bereitgestellt werden, deren Querschnittsform sowohl im gestauchten als auch im nicht gestauchten Zustand kreisförmig ist. Insbesondere tritt bei Formveränderung der Grundstruktur durch Stauchen eine zentrische Streckung der Querschnittsform ein. In anderen Worten wird durch Handhaben der Grundstruktur, z.B. durch Stauchen, die Winkelbeziehung zwischen LED und Strahlungsdetektor nicht beeinflusst. Somit bleibt die Orientierung zwischen LED und Strahlungsdetektor vorteilhaft auch bei unterschiedlichen Formveränderungen der Grundstruktur, beispielsweise durch Aufnahme eines kleinen Fingers oder eines großen Fingers, konstant. Folglich kann auf diese Weise sichergestellt werden, dass die Messeigenschaften des Sauerstoffsättigungssensors für jede Messstelle im Wesentlichen konstant und somit verbessert sind. Ein Beispiel einer derartigem Röhre ist auch als Extensionshülse bzw. als Fingerfalle (chinese finger trap) bekannt.

Besonders bevorzugt ist die Grundstruktur als gestrickte Röhre derart ausgebildet, dass durch Deformation der Grundstruktur der Querschnitt quer zur Längsachse rückstellend veränderlich ist.

Vorzugsweise ist die Röhre einwandig oder mehrwandig, beispielsweise doppelwandig ausgebildet.

Die einwandige Ausbildung bietet den Vorteil einfacher Fertigbarkeit. Die mehrwandige Ausbildung bietet den Vorteil erhöhter Rückstellkräfte.

Die mehrwandige Ausbildung kann beispielsweise durch mehrfache Stülpung der Röhre über sich selber erreicht werden. Optional können hierbei die durch die Stülpung gebildeten Stoßkanten gefügt werden, z.B. durch Schweißen.

Vorzugsweise ist die Grundstruktur einseitig offen oder beidseitig offen ausgebildet.

Bei einseitig offener Grundstruktur kann die Abschirmung des Inneren des Sauerstoffsättigungssensors gegen Streulicht vorteilhaft gegenüber der beidseitig offenen Grundstruktur verbessert werden.

Bei beidseitig offener Grundstruktur kann die Reinigbarkeit des Sauerstoffsättigungssensors gegenüber der einseitig offenen Grundstruktur verbessert werden, sofern der Sauerstoffsättigungssensor nicht als wegwerfbarer Sauerstoffsättigungssensor ausgestaltet ist.

Vorzugsweise kann der Sauerstoffsättigungssensor ferner eine lichtundurchlässige Struktur aufweisen, welche wenigstens einen Teil der Grundstruktur und/oder wenigstens einen Teil der Mehrzahl biegeelastischer Garne umgibt, um einen Lichteinfall aus der Umgebung des Sauerstoffsättigungssensors in Richtung des Strahlungsdetektors zu verringern.

Eine lichtundurchlässige Struktur bezeichnet eine Struktur, welche dazu ausgelegt ist, die Transmission von Licht durch die Grundstruktur zu verringern.

Auf diese Weise kann das Signal-Rausch-Verhalten des Sauerstoffsättigungssensors und somit dessen Messeigenschaften verbessert werden.

In einer bevorzugten Ausgestaltung kann die lichtundurchlässige Struktur als eine zweite, die Grundstruktur auf ihrer Außenseite wenigstens teilweise, vorzugsweise vollständig umgebende Schicht ausgestaltet sein.

Eine solche Struktur kann beispielsweise mit der Grundstruktur integral ausgebildet sein, beispielsweise in Form einer Beschichtung der Grundstruktur, oder als separate Schicht ausgestaltet sein, beispielsweise als Überzug der Grundstruktur, insbesondere als gestrickter textiler Überzug.

Eine weitere Möglichkeit eine lichtundurchlässige Struktur vorzusehen ist, die Grundstruktur zu einem Teil aus biegeelastischem Garn und zu einem anderen Teil aus lichtreduzierendem Garn herzustellen. Wenigstens ein Teil der Mehrzahl biegeelastischer Garne wird somit von lichtreduzierenden Garnen als lichtundurchlässige Struktur wenigstens einseitig umgeben. Beispielsweise kann hierzu in eine aus lichtreduzierendem Garn gestrickte Struktur eine Anzahl biegeelastischer Garne eingelegt sein oder die Grundstruktur kann aus biegeelastischem Garn und lichtreduzierendem Garn gewebt sein, beispielsweise indem ein Teil oder alle der Schussfäden oder Kettfäden durch das lichtreduzierende Garn gebildet werden.

Besonders bevorzugt ist die Grundstruktur als zweigeteilte gestrickte Röhre ausgebildet, welche eine erste innenliegende Röhre und eine zweite außenliegende Röhre aufweist. Die erste innenliegende Röhre kann dabei eine Mehrzahl von Garnen umfassen, welche einen guten Tragekomfort gewährleisten und die zweite außenliegende Röhre kann eine Mehrzahl von Garnen umfassen, welche eine lichtundurchlässige Struktur bereitstellen.

Vorzugsweise ist die LED und/oder der Strahlungsdetektor durch Schweißen, insbesondere Punktschweißen, Kleben, Einlegen oder Nähen in oder an der Grundstruktur aufgenommen.

Auf diese Weise können die LED und/oder der Strahlungsdetektor mit der Grundstruktur verbunden werden, ohne die Deformierbarkeit der Grundstruktur nachteilhaft zu beeinflussen.

Vorzugsweise sind die LED und/oder der Strahlungsdetektor als Teil einer oder mehrerer flexibler Leiterplatten in oder an der Grundstruktur aufgenommen.

Auf diese Weise können die LED und/oder der Strahlungsdetektor mit der Grundstruktur verbunden werden, ohne die Deformierbarkeit der Grundstruktur nachteilhaft zu beeinflussen.

Besonders bevorzugt sind die LED und/oder der Strahlungsdetektor als Teil einer oder mehrerer flexibler Leiterplatten durch Schweißen, insbesondere Punktschweißen, Kleben oder Nähen in oder an der Grundstruktur aufgenommen. In einer weiteren Variante ist die Leiterplatte in die Grundstruktur als Teil dieser eingeflochten oder eingewebt.

Vorzugsweise weist der Sauerstoffsättigungssensor ferner ein Fixiermittel auf, welches eingerichtet ist, den Sauerstoffsättigungssensor auf oder an der Messstelle zu fixieren.

Auf diese kann die Fixierkraft des Sauerstoffsättigungssensors auf der Messstelle weiter erhöht werden.

Das Fixiermittel kann beispielsweise als Band mit Klettverschluss oder als Gummiring ausgestaltet sein.

Der Sauerstoffsättigungssensor kann darüber hinaus weitere Elemente, zum Beispiel zur Datenverarbeitung und Kommunikation aufweisen. Beispielsweise kann der Sauerstoffsensor eine Steuereinheit wie eine Schaltung oder einen Mikroprozessor zur Steuerung der LED und des Strahlungsdetektors aufweisen. Die Steuereinheit kann beispielsweise ausgestaltet sein, Messsignale des Strahlungsdetektors zu empfangen und zu verarbeiten und/oder in einer Speichereinheit zu speichern. Ferner beispielsweise kann der Sauerstoffsättigungssensor Schaltkreise zur Ansteuerung und/oder zum Auslesen der LED und/oder des Strahlungsdetektors aufweisen. Die Schaltkreise können beispielsweise einen oder mehrere Verstärker, Widerstände, Kondensatoren, Filter, A/D-Wandler und dergleichen aufweisen. Die Schaltkreise können als Teil der Steuereinheit oder als separate Bauelemente ausgestaltet sein. Ferner beispielsweise kann der Sauerstoffsättigungssensor eine datentechnische Schnittstelle, beispielsweise eine kabelgebundene oder kabellose Schnittstelle, zum Senden und/oder Empfangen von Daten aufweisen. Beispielsweise können mittels der Schnittstelle die Messsignale des Strahlungsdetektors und/oder in einer Speichereinheit gespeicherte Messdaten an einen externen Empfänger übermittelt werden. Ferner beispielsweise kann der Sauerstoffsättigungssensor eine Energiequelle wie einen Akkumulator oder eine Batterie zur Bereitstellung elektrischer Energie für den Betrieb des Sauerstoffsättigungssensors aufweisen. Ergänzend oder alternativ kann der Sauerstoffsättigungssensor eine energietechnische (kabelgebundene oder kabellose) Schnittstelle zum Empfangen elektrischer Energie für den Betrieb des Sauerstoffsättigungssensors aufweisen. Alle vorgenannten Elemente können als Teil einer gemeinsamen Leiterplatte ausgestaltet sein, beispielsweise als Teil der vorbeschriebenen flexiblen Leiterplatte bzw. Leiterplatten.

Vorzugsweise weist der Sauerstoffsättigungssensor in Bezug auf seinen Einsatzzweck eine diesen indizierende Farbkennzeichnung auf. Beispielsweise kann ein wiederverwendbarer Sauerstoffsättigungssensor in einer ersten Farbe ausgestaltet sein und ein nicht wiederverwendbarer Sauerstoffsättigungssensor in einer zweiten, von der ersten Farbe verschiedenen Farbe ausgestaltet sein.

Diese und weitere Merkmale und Vorteile ergeben sich auch aus der nachstehenden Figurenbeschreibung. Dabei zeigt:
- **Fig. 1a**: ein Ausführungsbeispiel eines erfindungsgemäßen Sauerstoffsättigungssensors im Längsschnitt,
- **Fig. 1b**: ein Ausführungsbeispiel eines erfindungsgemäßen Sauerstoffsättigungssensors im Querschnitt durch die Querschnittsebene,
- **Fig. 2a**: ein Ausführungsbeispiel eines erfindungsgemäßen Sauerstoffsättigungssensors in Vorderansicht vor Verformung,
- **Fig. 2b**: ein Ausführungsbeispiel eines erfindungsgemäßen Sauerstoffsättigungssensors in Vorderansicht nach Verformung,
- **Fig. 3a**: ein weiteres Ausführungsbeispiel eines erfindungsgemäßen Sauerstoffsättigungssensors in Vorderansicht vor Verformung,
- **Fig. 3b**: ein weiteres Ausführungsbeispiel eines erfindungsgemäßen Sauerstoffsättigungssensors in Vorderansicht nach Verformung,
- **Fig. 4**: ein Ausführungsbeispiel einer erfindungsgemäßen gewebten Röhre in Detailansicht in Draufsicht,
- **Fig. 5**: ein Ausführungsbeispiel einer erfindungsgemäßen gewebten Röhre in Detailansicht im Längsschnitt,
- **Fig. 5b**: ein Ausführungsbeispiel einer erfindungsgemäßen gewebten Röhre in Detailansicht im Längsschnitt mit lichtundurchlässiger Struktur,
- **Fig. 5c**: ein weiteres Ausführungsbeispiel einer erfindungsgemäßen gewebten Röhre in Detailansicht im Längsschnitt mit lichtundurchlässiger Struktur,
- **Fig. 6**: ein weiteres Ausführungsbeispiel eines erfindungsgemäßen Sauerstoffsättigungssensors in Vorderansicht mit Fixiermittel,
- **Fig 7**: ein weiteres Ausführungsbeispiel eines erfindungsgemäßen Sauerstoffsättigungssensors in Vorderansicht im Längsschnitt.

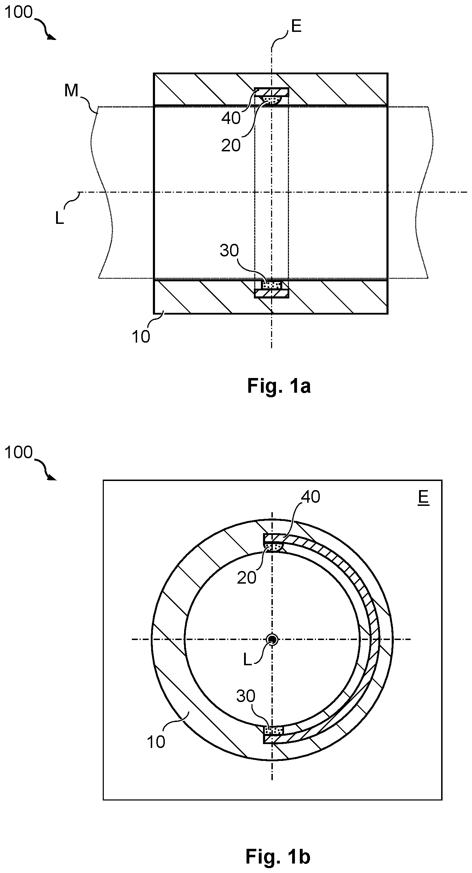

In Fig. 1a bis 3b und 6 - 7 sind Beispiele erfindungsgemäßer Sauerstoffsättigungssensoren 100 dargestellt.

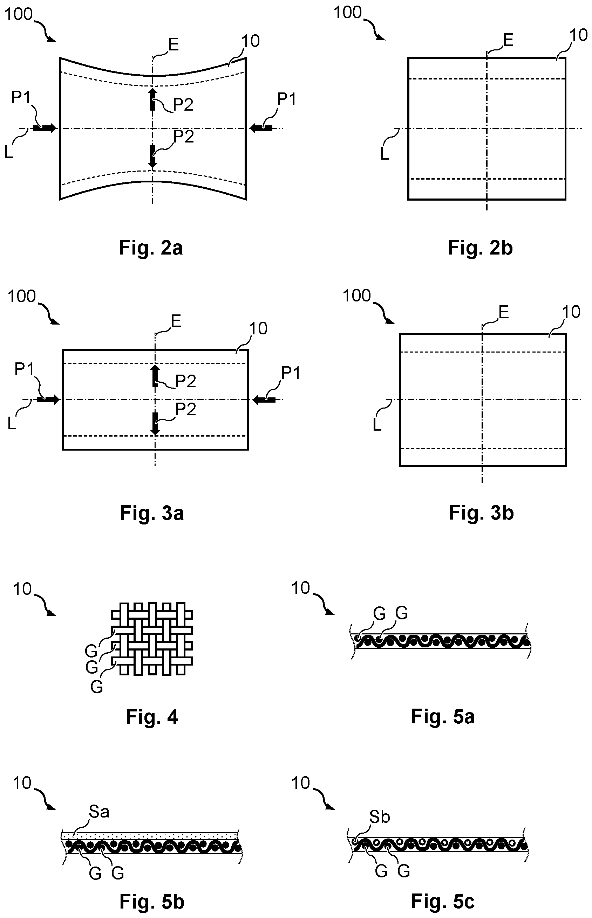

Jeder erfindungsgemäße Sauerstoffsättigungssensor 100 umfasst eine sich in Richtung einer Längsachse L erstreckende textile Grundstruktur 10. Die erfindungsgemäße Grundstruktur 10 kann beispielsweise zylinderförmig ausgestaltet sein, wie dies in den Ausführungsbeispielen nach Fig. 1a, 1b, 3a, 3b, 6 und 7 dargestellt ist. In einem anderen Ausführungsbeispiel ist die Grundstruktur 10 als Hyperboloid ausgestaltet, wie dies in Fig. 2a, 2b dargestellt ist. Weitere Formen der Grundstruktur 10 sind ebenfalls möglich.

Die Grundstruktur 10 jedes Ausführungsbeispiels weist eine rückstellend veränderliche Querschnittsform auf, so dass der Sauerstoffsättigungssensor 100 auf einer Messstelle M fixierbar ist. Die Messstelle M, welche beispielweise ein Finger eines Benutzers sein kann, ist in Fig. 1a schematisch angedeutet.

Es ist bevorzugt, dass die Querschnittsform der Grundstruktur 10 durch Stauchen der Grundstruktur 10 in Richtung der Längsachse L rückstellend veränderlich ist. Dies ist in den beiden Ausführungsbeispiel nach Fig. 2a und Fig. 3a angedeutet, welche jeweils einen Sauerstoffsättigungssensor 100 im Grundzustand, d.h. vor einer Deformation zeigen. Wird die Grundstruktur 10 entlang der Längsachse L gestaucht, d.h. deformiert, wie dies jeweils durch das Pfeilpaar P1-P1 angedeutet ist, verändert sich die Querschnittsform, insbesondere wird die Querschnittsform größer (vgl. Fig. 2b bzw. 3b im jeweils deformierten Zustand). In diesem Zustand kann der Sauerstoffsättigungssensor 100 auf die Messstelle M besonders schonend aufgesetzt werden. Durch Entlasten der rückstellend veränderlichen Grundstruktur 10 wird diese auf der Messstelle M durch Klemmkräfte fixiert.

Es ist alternativ in jedem Ausführungsbeispiel bevorzugt, dass die Querschnittsform durch Deformation der Grundstruktur 10 quer zur Längsachse L rückstellend veränderlich ist. Dies ist als Alternative in den beiden Ausführungsbeispiel nach Fig. 2a und Fig. 3a durch die Pfeilpaare P2-P2 angedeutet. Wird die Grundstruktur 10 quer zu der Längsachse L deformiert, wie dies jeweils durch das Pfeilpaar P2-P2 angedeutet ist, verändert sich die Querschnittsform, insbesondere wird die Querschnittsform größer (vgl. Fig. 2b bzw. 3b). Durch die aufgrund der Deformation wirkenden Rückstellkräfte wird die Grundstruktur 10 auf der Messstelle M durch Klemmkräfte fixiert.

Jeder erfindungsgemäße Sauerstoffsättigungssensor 100 umfasst ferner eine lichtemittierende Diode 20 (LED) und einen Strahlungsdetektor 30, wie dies in Fig. 1a und 1b dargestellt und in den übrigen Figuren der Übersichtlichkeit halber nicht dargestellt ist. Jeder erfindungsgemäße Sauerstoffsättigungssensor 100 kann mehr als eine LED 20 und/oder mehr als einen Strahlungsdetektor 30 aufweisen.

Die LED 20 und der Strahlungsdetektor 30 sind in oder an der Grundstruktur 10 aufgenommen und so in einer Querschnittsebene E der Grundstruktur 10 angeordnet, wie dies in Fig. 1a und 1b ersichtlich ist.

Die LED 20 und der Strahlungsdetektor 30 können, wie dies in dem Beispiel nach Fig. 1a, 1b dargestellt ist, mit ihrer äußeren Oberfläche bündig mit einer Begrenzungsfläche des durch den Grundkörper 10 bereitgestellten Hohlkörper angeordnet sein oder, in einer nicht dargestellten Ausgestaltung, über die Begrenzungsfläche des Hohlkörpers hinaus in diesen (teilweise oder vollständig) hineinragen, d.h. nicht bündig mit der Begrenzungsfläche sein.

Die Art und Weise der Aufnahme der LED 20 und des Strahlungsdetektors 30 in oder an dem Grundkörper 10 ist im Wesentlichen beliebig ausgestaltbar. Es ist jedoch in allen Ausführungsformen bevorzugt, dass die LED 20 und/oder der Strahlungsdetektor 30 (mittelbar oder unmittelbar) durch Schweißen, Kleben oder Nähen in oder an der Grundstruktur 10 aufgenommen sind. In einer weiteren Variante ist es bevorzugt, dass LED 20 und/oder Strahlungsdetektor 30 in dafür ausgestaltet Taschen der Grundstruktur 10 eingelegt sind.

Es ist ferner in allen Ausführungsbeispielen bevorzugt, dass die LED 20 und/oder der Strahlungsdetektor 30 als Teil einer oder mehrerer flexibler Leiterplatten 40 in oder an der Grundstruktur 10 aufgenommen sind, wie dies in Fig. 1a, 1b ersichtlich ist. In dem dargestellten Beispiel ist die Leiterplatte 40 in die Grundstruktur 10 eingebettet, es ist jedoch auch möglich, die Leiterplatte 40 bündig mit der Begrenzungsfläche des Hohlkörpers anzuordnen oder die Leiterplatte 40 auf einer Außenseite des Grundkörpers 10 anzuordnen. Die Leiterplatte 40 kann durch Schweißen, Kleben, Einlegen oder Nähen in oder an der Grundstruktur 10 aufgenommen sein, um die LED 20 und/oder den Strahlungsdetektor 30 mittelbar durch Schweißen, Kleben, Einlegen oder Nähen in oder an der Grundstruktur 10 aufzunehmen.

Es ist in erfindungsgemäß, dass die Grundstruktur 10 als geflochtene, gewickelte, gewebte und/oder gestrickte Röhre ausgebildet ist. Ein Beispiel einer gewebten Röhre ist in Fig. 4 als ausgebrochene Detailansicht in Draufsicht auf die Grundstruktur 10 dargestellt. Die gewebte Röhre wird durch eine Mehrzahl von Garnen G gebildet, welche gleichartig oder verschiedenartig ausgebildet sein können.

Obwohl das Gewebe nach Fig. 4 mit rechtwinklig zueinander verlaufenden Kettfäden und Schussfäden dargestellt ist, sind hiervon abweichende Winkelbeziehungen zwischen Kettfäden und Schussfäden bzw. zwischen den jeweils die Röhre bildenden Garnen möglich. Im Rahmen der Erfindung wurde insofern erkannt, dass die Verformbarkeit der Grundstruktur 10 vorteilhaft höher ist, je mehr der zwischen den beiden Garnrichtungen eingeschlossene Winkel von 90 ° abweicht. Insbesondere bei geflochtenen Röhren sind deutlich von 90 ° abweichende Winkel erzielbar.

Die gewebte Röhre nach Fig. 4 ist als abgebrochene Detailansicht im Längsschnitt in Fig. 5a dargestellt.

Es ist erfindungsgemäß, dass die Grundstruktur 10 eine Mehrzahl biegeelastischer Garne G umfasst, wobei wenigstens ein Teil der Garne G ausgewählt ist aus der Gruppe umfassend Kunststoffgarn, Glasgarn und Kohlenstoffgarn. Vorzugsweise ist das Material des Kunststoffgarns ausgewählt aus der Gruppe umfassend Polypropylen, Polyurethan und Polyethylen.

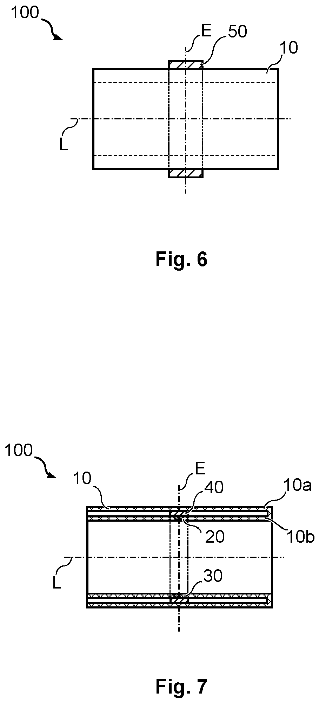

Die durch die Grundstruktur 10 gebildete Röhre kann, wie dies in Fig. 1a bis 3b dargestellt ist, einwandig ausgebildet sein. In Fig. 6 ist dargestellt, dass die durch die Grundstruktur 10 gebildete Röhre mehrwandig ausgebildet sein kann.

Ferner ist es in allen Ausführungsbeispielen möglich und in Fig. 1a bis 3a dargestellt, dass die Grundstruktur 10 beidseitig offen ausgebildet sein kann. Nicht dargestellt, aber in allen Ausführungsbeispielen möglich ist es, dass die Grundstruktur 10 einseitig offen ausgebildet ist. In diesem Fall kann eine der Stirnseiten der Grundstruktur 10 durch ein separates Element oder durch entsprechende geschlossen Ausbildung der textilen Grundstruktur 10 während des Herstellverfahrens verschlossen sein.

Es ist in allen Ausführungsbeispielen bevorzugt, dass der Sauerstoffsättigungssensor 100 eine lichtundurchlässige Struktur Sa, Sb aufweist, welche wenigstens einen Teil der Grundstruktur 10 und/oder wenigstens einen Teil der Mehrzahl biegeelastischer Garne G umgibt, um einen Lichteinfall aus der Umgebung des Sauerstoffsensors 100 in Richtung des Strahlungsdetektors 30 zu verringern.

Ein Ausführungsbeispiel einer derartigen lichtundurchlässigen Struktur Sa ist in Fig. 5b dargestellt. Dabei ist die lichtundurchlässige Struktur Sa als eine zweite, die Grundstruktur 10 wenigstens teilweise, vorzugsweise vollständig umgebende Schicht Sa ausgestaltet. Eine solche Schicht Sa kann beispielsweise als integraler Bestandteil der Grundstruktur 10, wie als Beschichtung, oder als separate Schicht, beispielsweise als Überzug vorgesehen sein.

Ein weiteres Ausführungsbeispiel einer lichtundurchlässigen Struktur Sb ist in Fig. 5c dargestellt. Dabei umfasst die Grundstruktur 10 sowohl biegeelastisches Garn G als auch lichtreduzierendes Garn Sb. Im dargestellten Beispiel ist die Röhre ein Gewebe, welche sowohl lichtreduzierende Garne Sb als auch biegeelastische Garne G umfasst.

In einigen Ausführungsbeispielen ist es bevorzugt, dass der Sauerstoffsättigungssensor 100 zusätzlich ein Fixiermittel 50 aufweist, welches eingerichtet ist, den Sauerstoffsättigungssensor 100 auf oder an der Messstelle M zu fixieren. Ein Beispiel eines derartigen Sauerstoffsättigungssensors 100 ist in Fig. 6 dargestellt. Das Fixiermittel 50 kann integral mit dem Sauerstoffsättigungssensor 100 verbunden sein, beispielsweise mit diesem vernäht sein. Alternativ kann das Fixiermittel 50 separat von dem Sauerstoffsättigungssensor 100 bereitgestellt sein und mit diesem zur Fixierung des Sauerstoffsättigungssensors 100 auf der Messstelle M mit diesem verbunden werden. Das Fixiermittel 50 kann beispielsweise ein Gummiring oder ein mit Klettverschluss versehenes Band sein, welches um den Sauerstoffsättigungssensor 100 geschlungen werden kann.

Es ist bevorzugt und in Fig. 6 dargestellt, dass die durch die Grundstruktur 10 gebildete Röhre, beispielsweise durch Stülpung der Röhre 10 über sich selber, mehrwandig ausgebildet sein kann. Hierdurch wird eine Grundstruktur 10 mit mehreren Wänden 10a, 10b erhalten. Eine derartige mehrwandige Grundstruktur 10 kann wie in Fig. 6 dargestellt dazu ausgebildet sein, die Leiterplatte 40 dazwischen aufzunehmen.

### Bezugszeichenliste

- 10: Grundstruktur
- 10a: erste Wand
- 10b: zweite Wand
- 20: lichtemittierende Diode
- 30: Strahlungsdetektor
- 40: Leiterplatte
- 50: Fixiermittel
- 100: Sauerstoffsättigungssensor

- E: Querschnittsebene
- G, Gs: Garn
- L: Längsachse
- M: Messstelle
- P1, P1: Pfeil
- Sa, Sb: lichtundurchlässige Struktur

## Patentansprüche

1. Sauerstoffsättigungssensor (100), umfassend
- eine sich in Richtung einer Längsachse (L) erstreckende textile Grundstruktur (10),
wobei die Grundstruktur (10) eine rückstellend veränderliche Querschnittsform aufweist, so dass der Sauerstoffsättigungssensor (100) auf einer Messstelle (M) fixierbar ist,
- eine lichtemittierende Diode (20), und
- einen Strahlungsdetektor (30),
wobei die lichtemittierende Diode (20) und der Strahlungsdetektor (30) in oder an der Grundstruktur (10) aufgenommen und so in einer Querschnittsebene (E) der Grundstruktur (10) angeordnet sind,
wobei die Grundstruktur (10) als geflochtene, gewickelte, gewebte und/oder gestrickte Röhre ausgebildet ist,
wobei die Grundstruktur (10) eine Mehrzahl biegeelastischer Garne (G) umfasst, wobei wenigstens ein Teil der Garne (G) ausgewählt ist aus der Gruppe umfassend Kunststoffgarn, Glasgarn und Kohlenstoffgarn.

2. Sauerstoffsättigungssensor (100) nach Anspruch 1,
wobei die Querschnittsform durch Stauchen der Grundstruktur (10) in Richtung der Längsachse (L) rückstellend veränderlich ist, oder
wobei die Querschnittsform durch Deformation der Grundstruktur (10) quer zur Längsachse (L) rückstellend veränderlich ist.

3. Sauerstoffsättigungssensor (100) nach Anspruch 1 oder 2,
wobei das Material des Kunststoffgarns ausgewählt ist aus der Gruppe umfassend Polypropylen, Polyurethan und Polyethylen.

4. Sauerstoffsättigungssensor (100) nach einem der Ansprüche 1 bis 3, wobei die Röhre einwandig oder mehrwandig ausgebildet ist.

5. Sauerstoffsättigungssensor (100) nach einem der vorherigen Ansprüche,
wobei die Grundstruktur (10) einseitig offen oder beidseitig offen ausgebildet ist.

6. Sauerstoffsättigungssensor (100) nach einem der vorherigen Ansprüche, ferner aufweisend eine lichtundurchlässige Struktur (Sa, Sb), welche wenigstens einen Teil der Grundstruktur (10) und/oder wenigstens einen Teil der Mehrzahl biegeelastischer Garne (G) umgibt, um einen Lichteinfall aus der Umgebung des Sauerstoffsensors (100) in Richtung des Strahlungsdetektors (30) zu verringern.

7. Sauerstoffsättigungssensor (100) nach einem der vorherigen Ansprüche, wobei die lichtemittierende Diode (20) und/oder der Strahlungsdetektor (30) durch Schweißen, Kleben, Einlegen oder Nähen in oder an der Grundstruktur (10) aufgenommen sind.

8. Sauerstoffsättigungssensor (100) nach einem der vorherigen Ansprüche, wobei die lichtemittierende Diode (20) und/oder der Strahlungsdetektor (30) als Teil einer oder mehrerer flexibler Leiterplatten (40) in oder an der Grundstruktur (10) aufgenommen sind.

9. Sauerstoffsättigungssensor (100) nach einem der vorherigen Ansprüche, ferner aufweisend ein Fixiermittel (50), welches eingerichtet ist, den Sauerstoffsättigungssensor (100) auf oder an der Messstelle (M) zu fixieren.
